# EUROPÄISCHE PATENTSCHRIFT

(11) **EP 2 393 817 B1**
(45) Veröffentlichungstag und Bekanntmachung des Hinweises auf die Patenterteilung: **27.03.2013**
(21) Anmeldenummer: 10701024.1
(22) Anmeldetag: 26.01.2010
(51) Int. Cl.: C07D 493/04

(54) **VERFAHREN ZUR HERSTELLUNG VON 2,6-DIOXABICYCLO-(3.3.0)-OCTAN-4,8-DION**
METHOD FOR PRODUCING 2,6-DIOXABICYCLO-(3.3.0)-OCTANE-4,8-DIONE
PROCÉDÉ POUR PRÉPARER DE LA 2,6-DIOXABICYCLO-(3.3.0)-OCTAN-4,8-DIONE

(30) Priorität: 06.02.2009 DE 102009000661; 03.06.2009 DE 102009026673
(43) Veröffentlichungstag der Anmeldung: 14.12.2011
(73) Patentinhaber: Evonik Degussa GmbH, 45128 Essen (DE)
(72) Erfinder: DINGERDISSEN, Uwe, 64342 Seeheim (DE); PFEFFER, Jan, 45355 Essen (DE); TACKE, Thomas, 63755 Alzenau (DE); HAAS, Thomas, 48161 Münster (DE); SCHMIDT, Harald, 45481 Mülheim an der Ruhr (DE); VOLLAND, Michael, 48249 Dülmen (DE); RIMBACH, Michael, 44649 Herne (DE); LETTMANN, Christian, 48653 Coesfeld (DE); SHELDON, Roger, NL-6997 AZ Hoog-Kappel (NL); JANSSEN, Michiel, NL-2497 DR Den Haag (NL)
(86) Internationale Anmeldenummer: PCT/EP2010/050832
(87) Internationale Veröffentlichungsnummer: WO 2010/089223

(56) Entgegenhaltungen:
- US-A- 4 543 168
- US-A- 5 136 103
- US-B1- 7 030 279
- HEYNS, KURT ET AL: "Selektive katalytische Oxydation von 1,4:3,6-Dianhydro-hexiten" CHEMISCHE BERICHTE, CODEN: CHBEAM; ISSN: 0009-2940, Bd. 96, Nr. 12, 1963, Seiten 3195-3199, XP002579295 in der Anmeldung erwähnt

## Beschreibung

Die vorliegende Erfindung betrifft ein Verfahren zur Herstellung von (2,6-Dioxabicyclo-(3.3.0)-octan-4,8-dion) **(I)** 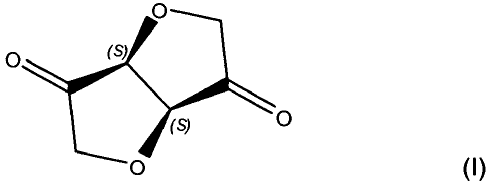 aus günstig zugänglichen Anhydro-Zuckern, auch Dianhydrohexitole genannt, wie 1,4:3,6-Dianhydro-D-Mannitol **(II),** 1,4:3,6-Dianhydro-D-Glucitol (Isosorbit), **(III)** und 1,4:3,6-Dianhydro-L-Iditol **(IV).**

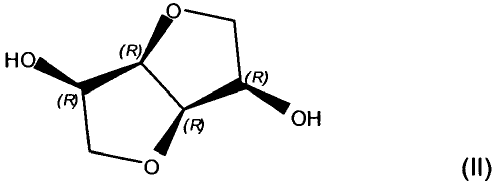

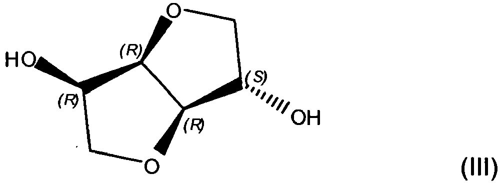

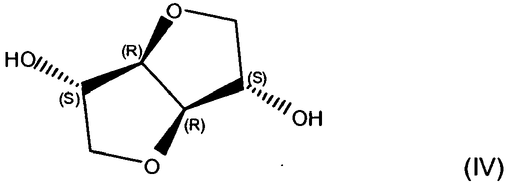

(2,6-Dioxabicyclo-(3.3.0)-octan-4,8-dion) **(I),** auch D-threo-2,5-Hexodiulose oder 1,4:3,6-dianhydro- (7Cl,8Cl,9Cl)-(Furo[3,2-b]furan) genannt, ist ein wichtiger Synthesebaustein und kann aus natürlichen Quellen gewonnen werden.

Die Oxidation von Zuckerbausteinen, wie L-Sorbose, D-Glucitol und D-Fructose zu 2,6-Dioxabicyclo-(3.3.0)-octan-4,8-dion wird z. B. bei G. C. Whiting und R. A. Coggins (Chemistry & Industry, 1963 (49), 1925-1926) als stöchiometrische Umsetzung mit Brom als oxidierendes Agens beschrieben. Ebenso gelingt die Umsetzung von (Poly)sachariden mit einem Überschuss von Natriumnitrit als Oxidans zu den entsprechenden oxidierten Verbindungen (T. J. Painter et al., Carbohydrate Res. 55, 95-103 (1977), ibid. 140, 61-68 (1985).

Neben den stöchiometrischen Umsetzungen sind auch katalytische Umsetzungen beschrieben.
Die Oxidation der endo-ständigen Hydroxygruppen des 1,4:3,6-Dianhydrohexitol in Gegenwart von kolloidalem Platin als Katalysator beschreiben Heynes et al. in Chemische Berichte (1963) 96(12), 3195 - 3199 als auch in der deutsche Offenlegungsschrift DE 14 43 385. Bei der Oxidation von 1,4:3,6-Dianhydro-D-Mannitol **(II)** erhält man das Diketon **(I).** Hingegen wird bei der Oxidation von 1,4:3,6-Dianhydro-D-Glucitol (Isosorbit)c **(III)** nur eine der Hydroxygruppen oxidiert, so dass ein Hydoxyketon (4-hydroxy-2,6-dioxabicyclo (3.3.0) octan-8-on [1S,4S,5R]) **(V)** entsteht:

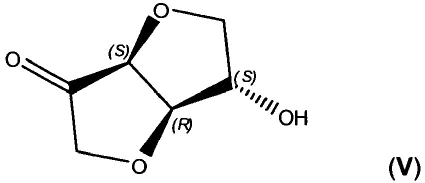

Die Oxidation von 1,4:3,6-Dianhydro-L-Iditol **(IV)** gelingt mittels dieses Verfahrens jedoch nicht. Keine von beiden OH-Gruppen lässt sich oxidieren. Folgerichtig weist Heynes daraufhin, dass die Umsetzung von stark gehinderten, d. h. exo-ständigen OH-Gruppen mit dieser Methode nicht gelingt.

Eine andere Methode dieser Oxidation beschreiben Jacquet et al. in Journal of Chemical Technology and Biotechnology (1990) 48(4), 493 - 506. Auch hier gelingt nur die Umsetzung des 1,4:3,6-Dianhydro-D-Glucitol (Isosorbit) **(III)** zum Hydoxyketon (4-hydroxy-2,6-dioxabicyclo (3.3.0)-octan-8-on [1S,4S,5R]) **(V).**

Die elektrochemische Oxidation von 1,4:3,6-Dianhydro-D-Mannitol **(II)** und 1,4:3,6-Dianhydro-D-Glucitol (Isosorbit) **(III)** mit Natriumbromid als Elektrolyt beschreibt die US-Patentschrift US 4,543,168. Auch hier konnte 1,4:3,6-Dianhydro-D-Mannitol **(II)** teilweise zum Diketon **(I)** oxidiert werden, wobei hingegen beim 1,4:3,6-Dianhydro-D-Glucitol (Isosorbit) **(III)** nur die endoständige Hydroxygruppe zum Hydroxyketon **(V**) oxidiert werden konnte.

US 7,030,279 beschreibt allgemein die Oxidation von primären oder sekundären Alkoholen mit Sauerstoff als Oxidans zu den korrespondierenden Aldehyden und Ketonen mittels eines Katalysatorsystems bestehend aus einem freien Nitroxylradikalderivat, einer Nitratquelle, einer Bromquelle und einer Carbonsäure, wobei die Carbonsäure in allen Fällen Essigsäure ist.

Xinquan Hu et al. beschreiben in J. AM. CHEM. SOC. 2004, 126, 4112-4113 die Alkohol-Oxidation mittels Nitroxylradikalderivat und Sauerstoff, wobei die Nitratquelle durch eine Nitritquelle ersetzt wurde. Hierbei wird allerdings explizit ausgeführt, dass auf den Einsatz einer Bromquelle nicht verzichtet werden kann. Das aufgeführte Lösungsmittel ist in den beschriebenen Beispielen Dichlormethan.

Die Verfahren gemäß dem Stand der Technik haben den Nachteil, dass sie aufgrund der geringen Ausbeuten und vielen Verfahrensschritten oder dem stöchiometrischem Einsatz von teuren und giftigen Reagenzien für eine großtechnische Herstellung von 2,6-Dioxabicyclo-(3.3.0)-octan-4,8-dion ungeeignet sind. Insbesondere die bei der Nitroxylradikal-katalysierten Umsetzung von Alkoholen benötigten Zusätze von Bromquellen in Verbindung mit Carbonsäuren oder in organischen Lösungsmitteln stellen äußerst korrosive Systeme dar, die eine Überführung in den technischen Maßstab erschweren.

Es war daher die Aufgabe der vorliegenden Erfindung, ein Verfahren zur Herstellung von 2,6-Dioxabicyclo-(3.3.0)-octan-4,8-dion **(I)** bereitzustellen, das die oben genannten Nachteile umgeht und ökonomisch vorteilhaft durchführbar ist.

Überraschenderweise wurde ein Verfahren zur Herstellung von 2,6-Dioxabicyclo-(3.3.0)-octan-4,8-dion **(I)** gefunden, wobei als Edukt der jeweilige Anhydrozucker 1,4:3,6-Dianhydro-D-Mannitol **(II),** 1,4:3,6-Dianhydro-D-Glucitol (Isosorbit), **(III)** oder 1,4:3,6-Dianhydro-L-Iditol **(IV)** eingesetzt wird. Darüber hinaus können aber auch die entsprechenden Hydroxyketone, insbesondere das Hydroxyketon **(V)** kann in dem erfindungsgemäßen Verfahren eingesetzt werden.

Gegenstand der Erfindung ist somit ein Verfahren zur Herstellung von 2,6-Dioxabicyclo-(3.3.0)-octan-4,8-dion **(I)** umfassend die Oxidation von Dianhydrohexitolen **(II-IV),** oder von entsprechenden Hydroxyketonen, insbesondere von Hydroxyketon **(V)** zur Hexodiulose **(I)** mit einem sauerstoffhaltigen Gas in Gegenwart einer Katalysatorzusammensetzung, umfassend mindestens ein Nitroxylradikal, eine oder mehrere NO-Quellen, wie z. B. Nitrat, Nitrit oder ein Stickoxid-Gas, und mindestens eine oder mehrere Carbonsäuren oder deren Anhydride und/oder Mineralsäuren oder deren Anhydride, gegebenenfalls in Gegenwart eines oder mehrerer Lösungsmittel, wobei die Umsetzung ohne Zusatz von Halogenquellen erfolgt.

Das erfindungsgemäße Verfahren hat den Vorteil, dass der Anhydrozucker mit einer milden Methode in Gegenwart von Nitroxylradikalen in einem einzigen Verfahrensschritt oxidiert wird. Mit dieser milden Methode können überraschenderweise neben den endo-ständigen auch die exo-ständigen sekundären Hydroxygruppen der bicyclisch cis-verknüpften fünfgliedrigen Ringsysteme zu Ketogruppen oxidiert werden. Dabei kann, insbesondere bei Einsatz von 1,4:3,6-Dianhydro-D-Glucitol **(III)** als Edukt, ein quantitativer Umsatz erzielt werden, wobei das Diketon **(I)** mit einer Selektivität von bis zu 100 %, gegebenenfalls neben kleineren Mengen des Hydroxyketons **(V)** gebildet wird. Entsprechende Umsätze gelingen auch mit 1,4:3,6-Dianhydro-D-Mannitol **(II)** und gegebenenfalls auch mit 1,4:3,6-Dianhydro-L-Iditol **(IV).** Insbesondere gelingt es, korrosive Halogenquellen zu vermeiden und das Substrat, das die NO-Quelle darstellt, den jeweiligen Prozessanforderungen durch Variation so anzupassen, dass eine einfache Aufarbeitung des Reaktionsaustrags gewährleistet ist. Von besonderem Vorteil ist aber, dass das Verfahren ohne Zusatz von externen Halogenquellen, also halogenfrei erfolgt. Halogenquellen im Sinne der vorliegenden Erfindung meint alle halogenhaltigen Verbindungen, die Halogene in elementarer Form oder halogenhaltige Ionen in beliebiger Oxidationsstufe freisetzen können. Ein Zusatz halogenhaltiger Verbindungen, wie Chlorite oder bromhaltige Verbindung wie *N*-Bromsuccinimid, *N*-Bromphthalimid, Tetrabutylammoniumbromid oder anorganische Salze, wie beispielsweise NH₄Br, Alkali- oder Erdalkalibromide zur Durchführung der Oxidation, kann, anders als im Stand der Technik, also entfallen.

Daraus ergibt sich, dass auch das erhaltene Verfahrensprodukt weitestgehend halogenfrei ist, wobei halogenfrei im Sinne der vorliegenden Erfindung meint, dass kein Halogen aus einer externen Halogenquelle stammt.

In dem erfindungsgemäßen Verfahren werden bevorzugt Dianhydrohexitole, ausgewählt aus 1,4:3,6-Dianhydro-D-Mannitol **(II),** 1,4:3,6-Dianhydro-D-Glucitol (Isosorbit) **(III)** und 1,4:3,6-Dianhydro-L-Iditol **(IV)** eingesetzt. Besonders bevorzugt wird als Edukt 1,4:3,6-Dianhydro-D-Glucitol (Isosorbit) **(III)** eingesetzt.

Die Oxidation des erfindungsgemäßen Verfahrens wird mit einer Katalysatorzusammensetzung in Abwesenheit von Übergangsmetallen durchgeführt.

Wesentlicher Bestandteil der im erfindungsgemäßen Verfahren eingesetzten Katalysatorzusammensetzung sind die Nitroxylradikale. Im Rahmen dieser Erfindung werden unter Nitroxylradikalen Verbindungen verstanden, die die Atomgruppierung 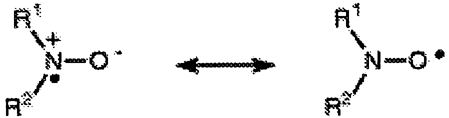 enthalten und die bei Raumtemperatur in Gegenwart von Sauerstoff mindestens eine Woche stabil sind. Diese Nitroxylradikale weisen am α-C-Atom benachbart zum Stickstoffatom keine Wasserstoffatome auf.

In dem erfindungsgemäßen Verfahren werden als Nitroxylradikale in der Katalysatorzusammensetzung bevorzugt Verbindungen gemäß der Struktur **(VI)** und/oder salzartige Verbindungen gemäß der der Struktur **(VII)** eingesetzt: 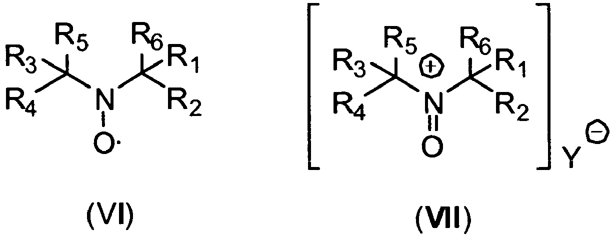 mit:
R¹, R², R³, R⁴, R⁵ und R⁶ = (C₁-C₁₀)-Alkyl-, (C₁-C₁₀)-Alkenyl-, (C₁-C₁₀)-Alkoxy-, (C₆-C₁₈)-Aryl-, (C₇-C₁₉)-Aralkyl-, (C₆-C₁₈)-Aryl-(C₁-C₈)-Alkyl- oder (C₃-C₁₈)-Heteroarylgruppe,
wobei die Substituenten vom Typ R¹, R², R³, R⁴, R⁵ und R⁶ gleich oder verschieden sind und die Substituenten vom Typ R⁵ und R⁶ zusammen eine (C₁-C₄)-Alkylenbrücke bilden können, die gesättigt oder ungesättigt, unsubstituiert oder substituiert sein kann, insbesondere mit einem oder mehreren Substituenten ausgewählt aus R¹, C₁-C₈-Amido-, Halogen, Oxy-, Hydroxy-, Amino-, Alkylamino-, Dialkylamino-, Arylamino-, Diarylamino-, Alkylcarbonyloxy-, Arylcarbonyloxy-, Alkylcarbonylamino- und Arylcarbonylaminogruppe.
In der Struktur **(VII)** ist Y⁻ ein beliebiges halogenfreies Anion.

In dem erfindungsgemäßen Verfahren kann sowohl ein Nitroxylradikal, es kann aber auch eine Mischung aus verschiedenen Nitroxylradikalen eingesetzt werden.

Vorzugsweise werden in dem erfindungsgemäßen Verfahren als Nitroxylradikale 2,2,6,6-Tetramethylpiperidin-1-oxyl (TEMPO) und/oder die an Position 4 des Heterocyclus substituierten Derivate des 2,2,6,6-Tetramethylpiperidin-1-oxyls eingesetzt, wobei die Derivate einen oder mehrere Substituenten ausgewählt aus R¹, C₁-C₈-Amido-, Halogen, Oxy-, Hydroxy-, Amino-, Alkylamino-, Dialkylamino-, Arylamino-, Diarylamino-, Alkylcarbonyloxy-, Arylcarbonyloxy-, Alkylcarbonylamino- und Arylcarbonylaminogruppen aufweisen, worin R¹ eine (C₁-C₁₀)-Alkyl-, (C₁-C₁₀)-Alkenyl-, (C₁-C₁₀)-Alkoxy-, (C₆-C₁₈)-Aryl-, (C₇-C₁₉)-Aralkyl-, (C₆-C₁₈)-Aryl-(C₁-C₈)-Alkyl- oder (C₃-C₁₈)-Heteroarylgruppe bedeutet. Beispiele entsprechender Verbindungen sind 4-Methoxy-2,2,6,6-tetramethylpiperidin-1-oxyl (4-MeO-TEMPO), 4-Oxo-2,2,6,6-tetramethylpiperidin-1-oxyl (4-oxo-TEMPO), 4-Hydroxy-2,2,6,6-tetramethylpiperidin-1-oxyl (4-hydroxy-TEMPO), 4-Benzoyloxy-2,2,6,6-tetramethylpiperidin-1-oxyl (BnO-TEMPO), 4-Acetamido-2,2,6,6-tetramethylpiperidin-1-oxyl, 4-Acetamino-2,2,6,6-tetramethylpiperidin-1-oxyl (AA-TEMPO), 4-Amino-2,2,6,6-tetramethylpiperidin-1-oxyl, N,N-Dimethylamino-2,2,6,6-tetramethyl-piperidin-1-oxyl (NNDMA-TEMPO), 3,6-Dihydro-2,2,6,6-tetramethyl-1 (2H)-pyridinyl-oxyl (DH-TEMPO) oder Bis-(2,2,6,6-tetramethylpiperidin-1-oxyl-4-yl) sebacat, wobei die genannten Beispiele einen oder mehrere Substituenten, ausgewählt aus R¹, C₁-C₈-Amido-, Halogen, Oxy-, Hydroxy-, Amino-, Alkylamino-, Dialkylamino-, Arylamino-, Diarylamino-, Alkylcarbonyloxy-, Arylcarbonyloxy-, Alkylcarbonylamino- und Arylcarbonylaminogruppe, aufweisen können.

Das Tetramethylpiperidin-N-oxyl-Strukturfragment gemäß den Strukturen **(VI)** oder **(VII)** kann auch ein Bestandteil eines größeren Makromoleküls, eines Oligomers oder auch einer Polymerstruktur sein. Ein Beispiel für ein solches Nitroxylradikal zeigt die Struktur **(VIII):**

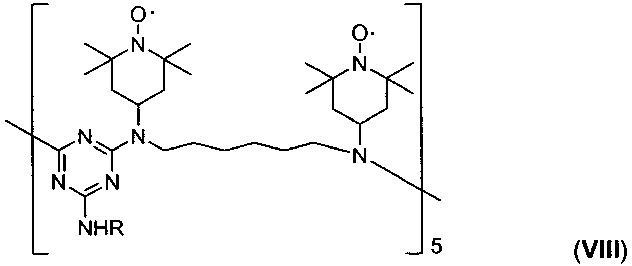

Die Nitroxylradikale können auch in heterogener Form in dem erfindungsgemäßen Verfahren eingesetzt werden, dies bedeutet dass die Nitroxylradikale auf einen Träger, beispielsweise Aluminiumoxid, Siliziumdioxid, Titandioxid oder Zirkoniumdioxid, aufgetragen sind. Als Trägermaterial für das Nitroxylradikal sind auch Polymere, Verbundstoffe oder Kohlenstoff einsetzbar. In einer anderen Ausführungsform können die Nitroxylradikale auch in immobilisierter Form z. B. in Gelpartikeln, Kapseln oder Polymermatrices vorliegen.

Bevorzugt werden in dem erfindungsgemäßen Verfahren die oben genannten Verbindungen AA-TEMPO, 4-Hydroxy-TEMPO, TEMPO und 4-Oxo-TEMPO als Nitroxylradikale eingesetzt. Besonders bevorzugt werden AA-TEMPO, 4-Hydroxy-TEMPO und TEMPO, insbesondere das AA-TEMPO, eingesetzt.

In dem erfindungsgemäßen Verfahren beträgt der Anteil an Nitroxylradikal vorzugsweise 0,001 bis 10 mol-%, bevorzugt 0,01 bis 5 mol-% und besonders bevorzugt 0,1 bis 2 mol-%, bezogen auf die Menge des eingesetzten Dianhydrohexitols.

Weiterhin enthält die in dem erfindungsgemäßen Verfahren eingesetzte Katalysatorzusammensetzung mindestens eine NO-Quelle. Als NO-Quelle können in dem erfindungsgemäßen Verfahren beispielsweise Salpetersäure, salpetrige Säure, Ammoniumnitrat oder -nitrit, oder Alkali- oder Erdalkalinitrate oder -nitrite, wie beispielsweise Magnesiumnitrat, oder Natriumnitrit eingesetzt werden. Zudem können in Ergänzung oder als Ersatz zu den Nitraten oder Nitriten stickoxidhaltige Gase wie N₂O, NO, N₂O₃. NO₂, N₂O₄ und N₂O₅ eingesetzt werden. Als NO-Quelle können auch Mischungen verschiedener, der oben genannten NO-Quellen eingesetzt werden. In dem erfindungsgemäßen Verfahren beträgt der Anteil der eingesetzten NO-Quelle(n) 0,001 bis 10 mol-%, bevorzugt von 0,01 bis 5 mol-% und ganz besonders bevorzugt von 0,1 bis 2 mol-%, bezogen auf die Menge des eingesetzten Dianhydrohexitols.

Weiterhin enthält die in dem erfindungsgemäßen Verfahren eingesetzte Katalysatorzusammensetzung mindestens eine oder mehrere Carbonsäuren oder deren Anhydride und/oder Mineralsäuren oder deren Anhydride.
Als Carbonsäure oder Carbonsäureanhydrid wird in dem erfindungsgemäßen Verfahren vorzugsweise Essigsäure oder Essigsäureanhydrid, Propionsäure oder eine andere Carbonsäure oder ein anderes Anhydrid, die sich in dem Reaktionsgemisch löst, eingesetzt. Bevorzugt wird in dem erfindungsgemäßen Verfahren Essigsäure eingesetzt. Es können auch Mischungen verschiedener geeigneter Carbonsäuren oder Lösungen von Carbonsäuren in einem geeigneten Lösemittel eingesetzt werden. Bevorzugt beträgt der Anteil der mindestens einen Carbonsäure 0,1 bis 200 mol-%, besonders bevorzugt 10 bis 50 mol-%, bezogen auf die Menge an eingesetztem Dianhydrohexitol.

Im Sinne der vorliegenden Erfindung wird der Terminus "Mineralsäuren" als Sammelbezeichnung für alle anorganischen Säuren verwendet. Geeignete Mineralsäuren sind beispielsweise H₂CO₃, H₃PO₄, HNO₃, HNO₂, H₂SO₄, H₂SO₃, H₃BO₃ oder deren Anhydride oder Mischungen hieraus.

Als Oxidationsmittel wird in dem erfindungsgemäßen Verfahren ein sauerstoffhaltiges Gas eingesetzt. Als sauerstoffhaltiges Gas kann reiner Sauerstoff eingesetzt werden, es können jedoch auch Mischungen von Sauerstoff mit einem Inertgas oder Luft oder einem an der Reaktion beteiligten Gas eingesetzt werden. Geeignete Inertgase sind beispielsweise Stickstoff, Kohlendioxyd, Helium oder Argon. Als an der Reaktion beteiligte Gase können beispielsweise Stickoxide eingesetzt werden, die bereits vorab bei der Beschreibung der NO-Quellen genannt wurden. Der Partialdruck des Sauerstoffs beträgt bevorzugt 0,1 bis 100 bar, besonders bevorzugt 0,2 bis 50 bar.

Die Durchführung des erfindungsgemäßen Verfahrens kann in einem Lösungsmittel oder ohne Einsatz eines Lösungsmittels erfolgen:
In einer besonderen Ausführungsform des erfindungsgemäßen Verfahrens wird der Verfahrensschritt in Gegenwart eines Lösemittels durchgeführt. Hierbei werden vorzugsweise polare Lösemittel, insbesondere polare organische Lösungsmittel eingesetzt. Bevorzugt wird als Lösungsmittel Acetonitril, Tetrahydrofuran, Ethylacetat, Aceton, Diethylether, Methyl-tert.-butylether, tertiäre Alkohole wie tert.-Amylalkohol, tert.-Butylalkohol, Ester der Kohlensäure wie Dimethylcarbonat, Diethylcarbonat, Kohlenwasserstoffe, oder eine Mischung dieser Lösungsmittel eingesetzt. Es werden vorzugsweise 0,1 bis 70 Vol-%, bevorzugt 0,5 bis 60 Vol-% und ganz besonders bevorzugt 1 bis 50 Vol-% an Lösungsmittel, bezogen auf die eingesetzte Menge an Dianhydrohexitol, eingesetzt.

In einer weiteren bevorzugten Ausführungsform des erfindungsgemäßen Verfahrens wird kein zusätzliches Lösemittel eingesetzt. In diesem Fall dient die Carbonsäure oder die mineralische Säure nicht nur als Komponente der Katalysatorzusammensetzung sondern auch als Lösungsmittel, um die Reaktionsmischung homogen zu halten. Die hat den Vorteil, dass auf Einsatz brennbarer und gegebenenfalls gesundheitsschädlicher Lösungsmittel verzichtet werden kann, bzw. eine gesonderte Abtrennung des Lösungsmittels entfallen kann.

Im Folgenden wird die Durchführung des erfindungsgemäßen Verfahrens erläutert. Die Oxidation des erfindungsgemäßen Verfahrens wird vorzugsweise bei einer Temperatur von 0 bis 100 °C oder am Siedepunkt des Lösungsmittels durchgeführt.

Der Gesamtdruck bei der Oxidation im erfindungsgemäßen Verfahren beträgt vorzugsweise 1 bis 300 bar, bevorzugt 1 bis 50 bar.

Das Verfahren kann sowohl als Batch-, Semi-Batch- oder als kontinuierliches Verfahren durchgeführt werden. Des Weiteren ist das erfindungsgemäße Verfahren an keinen bestimmten Reaktortyp gebunden, vielmehr kann der Verfahrensschritt in einem Rührkessel, in einem Rohrreaktor, in einer Kesselkaskade, in einem Mikroreaktor oder einer Kombination dieser Reaktortypen durchgeführt werden. Dabei kann der Katalysator homogen oder in heterogener Form wie zuvor beschrieben vorliegen.

In einer Ausführungsform des erfindungsgemäßen Verfahrens wird zunächst das Dianhydrohexitol in einem geeigneten Lösungsmittel gelöst oder suspendiert, anschließend wird die Katalysatorzusammensetzung dieser Lösung oder Suspension einzeln oder als Mischung zugegeben. Anschließend werden Druck und Temperatur eingestellt. Es ist jedoch auch möglich die Katalysatorzusammensetzung vorzulegen und die Lösung oder Suspension des Dianhydrohexitols der Katalysatorzusammensetzung zuzugeben. Im Falle einer kontinuierlichen Verfahrensführung wird vorzugsweise der Alkohol mit den Reaktionsgasen in der Ausführung eines Rieselbettes zugeführt.

In einer bevorzugten Ausführungsform des erfindungsgemäßen Verfahrens kann die Reaktion durch die Entfernung des Reaktionswassers aus dem Reaktionsgemisch beschleunigt werden. Hierfür werden der Reaktionsmischung wasserabsorbierende Mittel zugesetzt, vorzugsweise Natriumsulfat, Calciumoxid und Molekularsiebe, z. B. Zeolithe. Beispielsweise können die oben genannten Mineralsäuren bzw. deren Anhydride als Wasser bindende Säuren eingesetzt werden. Es ist weiterhin möglich, sehr stark Wasser komplexierende Lösungsmittel, wie z. B. Eisessig einzusetzen. Darüber hinaus ist es auch möglich Lösungsmittel einzusetzen, die Wasser chemisch binden können. Vorzugsweise handelt es sich dabei um die Anhydride der im erfindungsgemäßen Verfahren eingesetzten Carbonsäuren. Somit wirken die Carbonsäureanhydride nicht nur als Lösungsmittel für die Reaktion, sondern gleichzeitig auch als Wasser bindendes Mittel. Auf diese Weise wird eine effiziente Reaktionsführung ermöglicht, die gleichzeitig zu hohen Ausbeuten führt, ein Effekt, der in dieser Weise aus dem Stand der Technik nicht bekannt ist.
Wahlweise kann das Reaktionswasser aus der Reaktionsmischung auch durch destillative Entfernung oder durch extraktive Entfernung aus den Reaktionsgeschehen abgezogen werden.

Die Aufarbeitung der Reaktionsmischung geschieht in der Regel in Abhängigkeit von der Polarität der Zielmoleküle und der Löslichkeit der Nitroxylradikale. Im Falle des 2,6-Dioxabicyclo-(3.3.0)-octan-4,8-dions, welches sehr gut wasserlöslich ist und in Wasser in seiner Dihydratform vorliegt, bietet sich folgende Vorgehensweise an:
a) Entfernen der Lösungsmittel und der Carbonsäure durch Destillation oder Extraktion
b) Extraktion und gegebenenfalls Rezyklierung der Nitroxylradikale
c) Entfernung der gegebenenfalls aus den NO-Quellen stammenden Salze durch Ionentauscher, Elektrodialyse, Umkehrosmose, Membranenverfahren oder Ultrafiltration
d) Und/oder Reinigung des Produkts durch Kristallisation, Destillation, Extraktion und/oder chromatographische Trennung.
Die genannten Verfahrensschritte können dabei allein oder in beliebiger Kombination zueinander durchgeführt werden.

Das nach dem erfindungsgemäßen Verfahren erhaltene 2,6-Dioxabicyclo-(3.3.0)-octan-4,8-dion **(I)** kann in vorteilhafter Weise als Zwischenprodukt zur weiteren Umsetzung eingesetzt werden. So kann das Diketon beispielsweise durch reduktive Aminierung mit Ammoniak, Hydroxylamin oder Hydrazin in die entsprechenden Amine bzw. Diamine überführt werden, die vorteilhaft als Rohstoff für die Kunststoffherstellung eingesetzt werden können. Alternativ kann das erhaltene 2,6-Dioxabicyclo-(3.3.0)-octan-4,8-dion **(I)** hydrocyaniert werden, d. h. Cyanwasserstoff kann Säure-katalysiert an die Ketogruppen addiert werden. Die erhaltenen Additionsprodukte können in α-Hydroxycarbonsäuren, α-Aminocarbonsäuren (Aminosäuren), ungesättigte Carbonsäuren oder Amine umgewandelt werden. Derartige Verfahren sind dem Fachmann bekannt und können in einfacher Weise durchgeführt werden.

Beispielsweise kann die reduktive Aminierung in einer Stufe oder auch in mehreren Stufen durchgeführt werden. Vorzugsweise wird die reduktive Aminierung in einer Stufe durchgeführt. Hierbei wird das 2,6-Dioxabicyclo-(3.3.0)-octan-4,8-dion **(I)** direkt in Anwesenheit von Ammoniak, Wasserstoff, einem Hydrierkatalysator und gegebenenfalls weiteren Zusätzen, wie beispielsweise Molekularsieb, und in An- oder Abwesenheit von organischen Lösungsmitteln aminierend hydriert.

Im Falle eines mehrstufigen Verfahrens wird die Hexodiulose in Gegenwart von Ammoniak zunächst in das entsprechende Diimin umgewandelt (Verfahrensschritt a)) und anschließend hydriert (Verfahrensschritt b)). Das in dem Verfahrensschritt b) eingesetzte Diimin kann sowohl als Reinsubstanz als auch als direktes Verfahrensprodukt aus Verfahrensschritt a) eingesetzt werden.

Um die Gleichgewichtseinstellung der Iminierung in dem Verfahrensschritt a) zu beschleunigen ist es zweckmäßig, einen sogenannten Iminierungskatalysator zu verwenden. Hierzu können in dem Verfahrensschritt a) des erfindungsgemäßen Verfahrens die nach dem Stand der Technik bekannten Iminierungskatalysatoren eingesetzt werden. Vorzugsweise werden Iminierungskatalysatoren, ausgewählt aus anorganischen oder organischen Ionenaustauschern, geträgerten Heteropolysäuren, aciden Metalloxiden, insbesondere Aluminiumoxid und Titandioxid, Sulfonsäuregruppen-enthaltenden Organopolysiloxanen und sauren Zeolithen, eingesetzt. Auch mit Mineralsäuren gehandelte Aktivkohlen können zum Einsatz kommen. Der in Verfahrensschritt a) eingesetzte Iminierungskatalysator kann in Form eines Suspensionskatalysators oder Festbettkatalysators vorliegen.

Der Iminierungskatalysator und der Hydrierkatalysator sind bevorzugt jeweils in einem separaten Reaktor angeordnet. Es ist jedoch auch möglich, den Iminierungskatalysator zusammen mit dem Hydrierkatalysator in demselben Reaktor anzuordnen. Es können auch sogenannte bifunktionelle Katalysatoren zum Einsatz kommen, die sowohl die Iminierung als auch die Hydrierung katalysieren. Beispiele für derartige Katalysatoren sind Hydrierkatalysatoren, die auf anorganische oder organische Trägermaterialien der zuvor genannten Gruppen aufgebracht sind.

Die Iminierung in dem Verfahrensschritt a) des erfindungsgemäßen Verfahrens wird vorzugsweise bei einer Reaktionstemperatur von 10 bis 150 °C, bevorzugt von 30 bis 130 °C und ganz besonders bevorzugt von 40 bis 100 °C durchgeführt.

Der Druck beträgt in dem Verfahrensschritt a) des erfindungsgemäßen Verfahrens vorzugsweise von dem Eigendruck der Reaktionsmischung bis 50 MPa, bevorzugt von 0,3 bis 50 MPa und besonders bevorzugt von 5 bis 30 MPa. Ganz besonders bevorzugt wird der Verfahrensschritt a) bei demselben Druck wie der Verfahrensschritt b) - die Hydrierung - durchgeführt.

In einer bevorzugten Ausführungsform des erfindungsgemäßen Verfahrens wird in Verfahrensschritt a) das Gleichgewicht der Iminierung in die Richtung des Diimins verschoben, indem das bei der Iminierung gebildete Wasser entfernt wird. Hierfür werden der Reaktionsmischung im Verfahrensschritt a) wasserabsorbierende Mittel zugesetzt, vorzugsweise Molekularsiebe, Calciumoxid und Natriumsulfat. Insbesondere eignen sich hierfür Molekularsiebe, bevorzugt Molekularsiebe mit einer Porenweite von 4 Angström.

Pro Mol eingesetztem 2,6-Dioxabicyclo-(3.3.0)-octan-4,8-dion **(I)** werden in dem Verfahrensschritt a) des erfindungsgemäßen Verfahrens vorzugsweise von 1 bis 500 mol, bevorzugt von 5 bis 200 mol Ammoniak eingesetzt.

Die Iminierung der Hexodiulose in dem Verfahrensschritt a) des erfindungsgemäßen Verfahrens erfolgt vorzugsweise mit flüssigem Ammoniak. Bevorzugt wird diese Iminierung ohne eine Zugabe weiterer Lösemittel durchgeführt. Es ist jedoch möglich den Verfahrensschritt a) auch in Gegenwart zusätzlicher Lösemittel durchzuführen. Geeignete Lösemittel sind hierfür einwertige Alkohole mit 1 bis 4 Kohlenstoffatomen, insbesondere Methanol sowie Ether, insbesondere Tetrahydrofuran, Methyl-*tert*.-butylether oder Dioxan. Auch der Einsatz von Mischungen der obengenannten Lösemittel ist möglich.

Als Hydrierkatalysatoren können in dem Verfahrensschritt b) des erfindungsgemäßen Verfahrens prinzipiell alle Katalysatoren eingesetzt werden, die in der Lage sind die Hydrierung von Nitril- und/oder Imingruppen mit Wasserstoff zu katalysieren. Besonders geeignet sind hierfür Nickel-, Kupfer-, Eisen-, Palladium-, Rhodium-, Ruthenium- und Cobaltkatalysatoren; ganz besonders sind Cobaltkatalysatoren geeignet. Zur Erhöhung der Aktivität, Selektivität und/oder der Standzeit können diese Hydrierkatalysatoren zusätzlich Dotierungsmetalle oder andere Modifizierungsmittel enthalten. Typische Dotierungsmetalle sind beispielsweise Mo, Fe, Ag, Cr, Ni, V, Ga, In, Bi, Ti, Zr und Mn sowie die seltenen Erden. Typische Modifizierungsmittel sind beispielsweise solche, die die Säure-Base-Eigenschaften dieser Hydrierkatalysatoren beeinflussen können, wie beispielsweise Alkali- und Erdalkalimetalle bzw. deren Verbindungen, insbesondere Mg- und Ca-Verbindungen sowie Phosphorsäure oder Schwefelsäure sowie deren Verbindungen.

Die Hydrierkatalysatoren können in dem Verfahrensschritt b) des erfindungsgemäßen Verfahrens in Form von Pulvern oder Formkörpern, wie beispielsweise Kugeln, Extrudaten, Ringen oder gepressten Pulvern, eingesetzt werden. Es können Vollkontakte, Katalysatoren vom Typ Raney oder Trägerkatalysatoren in dem erfindungsgemäßen Verfahren zum Einsatz kommen. Bevorzugt sind Katalysatoren vom Typ Raney und Trägerkatalysatoren. Geeignete Trägermaterialien dieser Hydrierkatalysatoren sind beispielsweise Kieselgur, Siliziumdioxid, Aluminiumoxid, Alumosilikate, Titandioxid, Zirkoniumdioxid, Aluminium-Silizium-Mischoxide, Magnesiumoxid und Aktivkohle. Das Aktivmetall kann in dem Fachmann bekannter Weise auf das Trägermaterial aufgebracht werden, wie beispielsweise durch Imprägnierung, Aufsprühen oder Fällung. Je nach Art der Katalysatorherstellung sind weitere dem Fachmann bekannte, Präparationsschritte notwendig, wie beispielsweise Trocknung, Calcinierung, Formgebung und Aktivierung. Zur Formgebung können optional weitere Hilfsstoffe wie z. B. Graphit oder Magnesiumstearat zugesetzt werden.

Der für die Hydrierung in dem Verfahrensschritt b) des erfindungsgemäßen Verfahrens erforderliche Wasserstoff kann dem Reaktor entweder im Überschuss, vorzugsweise mit bis zu 10000 Moläquivalenten bezogen auf das Diimin, zugeführt werden oder in solch einer Menge, dass sowohl der durch die Hydrierung verbrauchte Wasserstoff als auch der Anteil des Wasserstoffs, der im Produktstrom den Reaktor verlässt, nachgeführt wird. Bei einer kontinuierlichen Fahrweise des erfindungsgemäßen Verfahrens kann der Wasserstoff in dem Verfahrensschritt b) im Gleich- oder Gegenstrom zugeführt werden.

Die Hydrierung des Diimins in dem Verfahrensschritt b) des erfindungsgemäßen Verfahrens erfolgt vorzugsweise in flüssigem Ammoniak als Lösemittel. Pro mol Hexodiulose werden vorzugsweise von 1 bis 500 mol, bevorzugt von 5 bis 200 mol Ammoniak eingesetzt. Zweckmäßigerweise wird mindestens die Ammoniakmenge eingesetzt, die bei der Iminierung in Verfahrensschritt a) eingesetzt wurde. Der Anteil an Ammoniak kann auch vor der Hydrierung - dem Verfahrensschritt b) - durch Zugabe bzw. Entfernung von Ammoniak auf den gewünschten Wert erhöht bzw. erniedrigt werden.

Bevorzugt wird diese Hydrierung ohne eine Zugabe weiterer Lösemittel durchgeführt. Es ist jedoch möglich den Verfahrensschritt b) auch in der Gegenwart von Lösemittel, ausgewählt aus einwertigen Alkoholen mit 1 bis 4 Kohlenstoffatomen, insbesondere Methanol, und Ethern, insbesondere Tetrahydrofuran, Methyl-*tert*.-butylether oder Dioxan, durchzuführen. Auch der Einsatz von Mischungen der obengenannten Lösemittel ist möglich.

Die Hydrierung in Verfahrensschritt b) des erfindungsgemäßen Verfahrens wird vorzugsweise bei einer Temperatur von 20 bis 150 °C und bevorzugt von 40 bis 130 °C durchgeführt.

Bei einem Druck vorzugsweise von 0,3 bis 50 MPa und bevorzugt von 2 bis 35 MPa wird die Hydrierung in dem Verfahrensschritt b) des erfindungsgemäßen Verfahrens durchgeführt.

Das Reaktionsgemisch des Verfahrensschritt b) des erfindungsgemäßen Verfahrens kann gemäß dem Stand der Technik bekannten Verfahren aufgearbeitet werden. Insbesondere wird hierbei der Ammoniak, die möglicherweise eingesetzten Lösemittel abgetrennt und das Diamin isoliert.

Die ebenfalls mögliche Hydrocyanierung kann generell auf jede dem Fachmann bekannte Art erfolgen.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende Offenbarung aufzufassen.

Nachfolgend wird die vorliegende Erfindung anhand von Beispielen näher erläutert.

### Beispiele:

### 1. Oxidation von Isosorbit mit AA-Tempo/O₂ /Nitrit/Nitrat:

### Material:

| | | |
|---|---|---|
| Isosorbit (1,4:3,6-Dianhydro-D-sorbitol) | 0,1 mol | → 73,62 g/L |
| AA-TEMPO | 5 mol-% | → 5,365 g/L |
| NaNO₂ | 2 mol-% | → 2,568 g/L |
| Mg(NO₃)₂ x 6H₂O | 2 mol-% | → 0,712 g/L |
| Essigsäure (100 %) | 200 mL | |
| Dekan (interner Standard für Analytik) | 7g | → 35 g/L |

### Reaktionsbedingungen:

| | |
|---|---|
| Reaktionstemperatur: | T = 50 °C |
| Reaktionszeit: | t = 4h |
| Sauerstoff: | 10 bar |
| Reaktorvolumen | 450 mL |

### Durchführung:

Das Edukt, der Katalysator, die Salze und der interne Standard werden vorgelegt, in der Essigsäure (Zugabe der Essigsäure bis das Volumen von 200 ml erreicht ist) gelöst und in den Hochdruckreaktor überführt. Der Reaktor wird verschlossen und mit Stickstoff zweimal inertisiert. Anschließend wird die Lösung unter Rühren auf eine Temperatur von 50 °C aufgeheizt. Zum Start der Reaktion wird 10 bar Sauerstoff auf den Autoklaven aufgedrückt. Der Druck in dem Reaktor wird bei 10 bar konstant gehalten, wobei verbrauchter Sauerstoff durch eine geöffnete Sauerstoffversorgung bis zu einem Druck von 10 bar nachdosiert wird. Nach vier Stunden wird die Reaktion durch Inertisierung mit Stickstoff und Abkühlung des Systems abgebrochen.

### Ergebnis:

Es wird ein Umsatz von größer 97 % beobachtet bei einer Selektivität für das Diketon von 100 %.

### 2. Oxidation von Isosorbit mit AA-Tempo/O₂ /Nitrit:

### Material:

| | | |
|---|---|---|
| Isosorbit (1,4:3,6-Dianhydro-D-sorbitol) | 0,1 mol | → 73,10 g/L |
| AA-TEMPO | 5 mol-% | → 5,335 g/L |
| NaNO₂ | 2,5 mol-% | → 3,45 g/L |
| Essigsäure (100 %) | 200 mL | |

### Reaktionsbedingungen:

| | |
|---|---|
| Reaktionstemperatur: | T = 50 °C |
| Reaktionszeit: | t = 4 h |
| Sauerstoff: | 10 bar |
| Reaktorvolumen | 450 mL |

### Durchführung:

Das Edukt, der Katalysator, die Salze und der interne Standard werden vorgelegt, in der Essigsäure (Zugabe der Essigsäure bis das Volumen von 200 ml erreicht ist) gelöst und in den Hochdruckreaktor überführt. Der Reaktor wird verschlossen und mit Stickstoff zweimal inertisiert. Anschließend wird die Lösung unter Rühren auf eine Temperatur von 50 °C aufgeheizt. Zum Start der Reaktion wird 10 bar Sauerstoff auf den Autoklaven aufgedrückt. Der Druck in dem Reaktor wird bei 10 bar konstant gehalten, wobei verbrauchter Sauerstoff durch eine geöffnete Sauerstoffversorgung bis zu einem Druck von 10 bar nachdosiert wird. Nach vier Stunden wird die Reaktion durch Inertisierung mit Stickstoff und Abkühlung des Systems abgebrochen.

### Ergebnis:

Es wird ein Umsatz von größer 97 % beobachtet bei einer Selektivität für das Diketon von 100 %.

### 3. Oxidation von Isosorbit mit AA-Tempo/O₂ /Nitrat:

### Material:

| | | |
|---|---|---|
| Isosorbit (1,4:3,6-Dianhydro-D-sorbitol) | 0,1 mol | → 73,23 g/L |
| AA-TEMPO | 5 mol-% | → 5,450g/L |
| Mg(NO₃)₂ x 6H₂O | 9,3 mol-% | → 3,460 g/L |
| Essigsäure (100 %) | 200 mL | |
| Dekan (interner Standard für Analytik) | 7g | → 35 g/L |

### Reaktionsbedingungen:

| | |
|---|---|
| Reaktionstemperatur: | T=50 °C |
| Reaktionszeit: | t=4h |
| Sauerstoff: | 10 bar |
| Reaktorvolumen | 450 mL |

### Durchführung:

Das Edukt, der Katalysator, die Salze und der interne Standard werden vorgelegt, in der Essigsäure (Zugabe der Essigsäure bis das Volumen von 200 ml erreicht ist) gelöst und in den Hochdruckreaktor überführt. Der Reaktor wird verschlossen und mit Stickstoff zweimal inertisiert. Anschließend wird die Lösung unter Rühren auf eine Temperatur von 50 °C aufgeheizt. Zum Start der Reaktion wird 10 bar Sauerstoff auf den Autoklaven aufgedrückt. Der Druck in dem Reaktor wird bei 10 bar konstant gehalten, wobei verbrauchter Sauerstoff durch eine geöffnete Sauerstoffversorgung bis zu einem Druck von 10 bar nachdosiert wird. Nach vier Stunden wird die Reaktion durch Inertisierung mit Stickstoff und Abkühlung des Systems abgebrochen.

### Ergebnis:

Es wird ein Umsatz von größer 97 % beobachtet bei einer Selektivität für das Diketon von 100 %.

### 4. Oxidation von Isosorbit mit AA-Tempo/O₂ /Nitrit/Nitrat:

Katalysator Menge 50%; Salz Menge jeweils 10%

### Material:

| | | |
|---|---|---|
| Isosorbit (1,4:3,6-Dianhydro-D-sorbitol) | 0,1 mol | → 73,25 g/L |
| AA-TEMPO | 2,5 mol-% | → 2,70 g/L |
| NaNO₂ | 0,2 mol-% | → 0,255 g/L |
| Mg(NO₃)₂ x 6H₂O | 0,2 mol-% | → 0,085 g/L |
| Essigsäure (100 %) | 200 mL | |
| Dekan (interner Standard für Analytik) | 7g | → 35 g/L |

### Reaktionsbedingungen:

| | |
|---|---|
| Reaktionstemperatur: | T = 50 °C |
| Reaktionszeit: | t = 4 h |
| Sauerstoff: | 10 bar |
| Reaktorvolumen | 450 mL |

### Durchführung:

Das Edukt, der Katalysator, die Salze und der interne Standard werden vorgelegt, in der Essigsäure (Zugabe der Essigsäure bis das Volumen von 200 ml erreicht ist) gelöst und in den Hochdruckreaktor überführt. Der Reaktor wird verschlossen und mit Stickstoff zweimal inertisiert. Anschließend wird die Lösung unter Rühren auf eine Temperatur von 50 °C aufgeheizt. Zum Start der Reaktion wird 10 bar Sauerstoff auf den Autoklaven aufgedrückt. Der Druck in dem Reaktor wird bei 10 bar konstant gehalten, wobei verbrauchter Sauerstoff durch eine geöffnete Sauerstoffversorgung bis zu einem Druck von 10 bar nachdosiert wird. Nach vier Stunden wird die Reaktion durch Inertisierung mit Stickstoff und Abkühlung des Systems abgebrochen.

### Ergebnis:

Es wird ein Umsatz von ca. 47 % beobachtet bei einer Selektivität für das Diketon von 76 %.

**Tab.1 Zusammenstellung der Versuche im Hochdruckreaktor zur Oxidation von Isosorbit mit AA-Tempo. Reaktionstemperatur 50 °C, Reaktionszeit 4h, Sauerstoff 10 bar (Leitung offen)**

| Beispiel | Isosorbit [g/l] | AA-Tempo [g/l] | NaNO₂ [g/l] | Mg(NO₃)₂x6H₂O [g/l] | Umsatz [%] | Selektivität (Diketon) [%] |
|---|---|---|---|---|---|---|
| 1 | 73,62 | 5,365 | 2,568 | 0,712 | 98,4 | 100 |
| 2 | 73,10 | 5,335 | 3,45 | - | 97,8 | 100 |
| 3 | 73,23 | 5,450 | - | 3,460 | 97,9 | 100 |
| 4 | 73,25 | 2,70 | 0,255 | 0,085 | 46,7 | 76 |

### 5. Oxidation von Isosorbit mit AA-Tempo/O₂ /Nitrit/Nitrat:

### Material:

| | | |
|---|---|---|
| Isosorbit (1,4:3,6-Dianhydro-D-sorbitol) | 25 mmol | → 73,07 g/L |
| AA-TEMPO | 5 mol-% | → 5,35 g/L |
| NaNO₂ | 2 mol-% | → 2,55 g/L |
| Mg(NO₃)₂ x 6H₂O | 2 mol-% | → 0,70 g/L |
| Essigsäure (100 %) | 50 mL | |
| Dekan (interner Standard für Analytik) | 1,75g | → 35 g/L |

### Reaktionsbedingungen:

| | |
|---|---|
| Reaktionstemperatur: | T = 50 °C |
| Reaktionszeit: | t = 1,5 h |
| Sauerstoff: | 10 mL / min |

### Durchführung:

Das Edukt, der Katalysator, die Salze und der interne Standard werden vorgelegt, in der Essigsäure (Zugabe der Essigsäure bis das Volumen von 50 ml erreicht ist) gelöst und in einen Vierhalskolben mit Rückflusskühler, Thermometer, Gaseinleitungsfritte und Septum zur Probennahme überführt. Anschließend wird die Lösung unter Rühren auf eine Temperatur von 50 °C aufgeheizt. Zum Start der Reaktion wird Sauerstoff mit einer Durchflussrate von 10 mL / min über die Gaseinleitungsfritte in die Reaktionslösung eingeleitet. Die Reaktion wird über einen Zeitraum von 1,5 h gefahren, wobei zur Aufnahme der Kinetik in Abständen von 15 min eine Probe entnommen wird.

### Ergebnis:

Es wird ein Umsatz von ca. 97 % beobachtet bei einer Selektivität für das Diketon von 100 %.

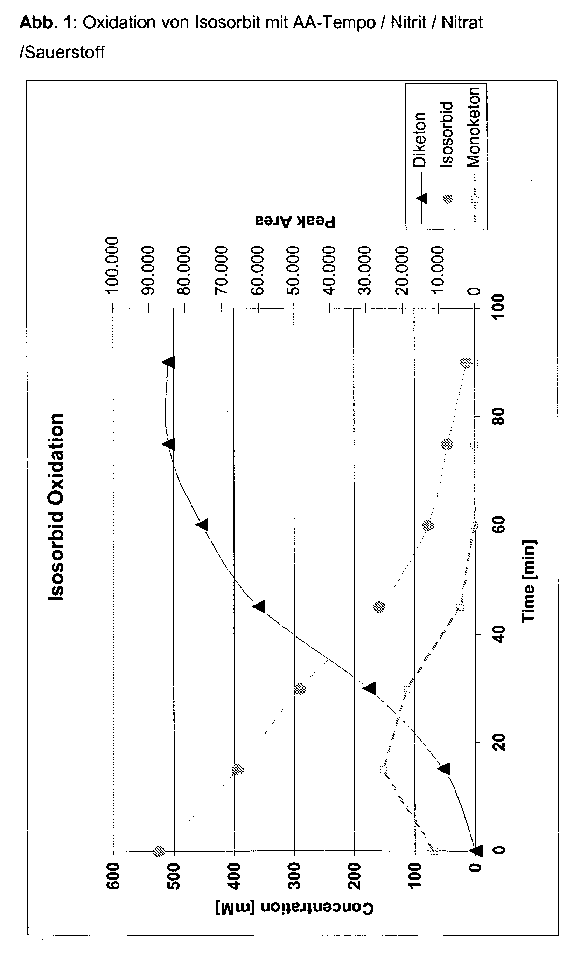

Abbildung 1 zeigt die Entwicklung der Isosorbit-, Diketon- und Monoketon-Konzentrationen in Abhängigkeit von der Zeit bei der Durchführung des erfindungsgemäßen Verfahrens. Dabei wurden die Isosorbit- und Diketon-Konzentrationen über Kalibrierkurven und dem internen Standard ausgewertet. Die Auswertung der Monoketon-Konzentrationen erfolgte über die Peakflächen.

### 6. Oxidation von Isosorbit mit AA-Tempo/O₂ /Nitrat:

### Material:

| | | |
|---|---|---|
| Isosorbit (1,4:3,6-Dianhydro-D-sorbitol) | 25 mmol | → 73,07 g/L |
| AA-TEMPO | 5 mol-% | → 5,35 g/L |
| Mg(NO₃)₂ x 6H₂O | 4 mol-% | → 1,40 g/L |
| Essigsäure (100 %) | 50 mL | |
| Dekan (interner Standard für Analytik) | 1,75g | → 35 g/L |

### Reaktionsbedingungen:

| | |
|---|---|
| Reaktionstemperatur: | T = 50 °C |
| Reaktionszeit: | t = 1,5 h |
| Sauerstoff: | 10 mL / min |

### Durchführung:

Das Edukt, der Katalysator, die Salze und der interne Standard werden vorgelegt, in der Essigsäure (Zugabe der Essigsäure bis das Volumen von 50 ml erreicht ist) gelöst und in einen Vierhalskolben mit Rückflusskühler, Thermometer, Gaseinleitungsfritte und Septum zur Probennahme überführt. Anschließend wird die Lösung unter Rühren auf eine Temperatur von 50 °C aufgeheizt. Zum Start der Reaktion wird Sauerstoff mit einer Durchflussrate von 10 mL / min über die Gaseinleitungsfritte in die Reaktionslösung eingeleitet. Die Reaktion wird über einen Zeitraum von 1,5 h gefahren, wobei zur Aufnahme der Kinetik in Abständen von 15 min eine Probe entnommen wird.

### Ergebnis:

Es wird ein Umsatz von ca. 36% beobachtet bei einer Selektivität für das Diketon von ca. 85 %.

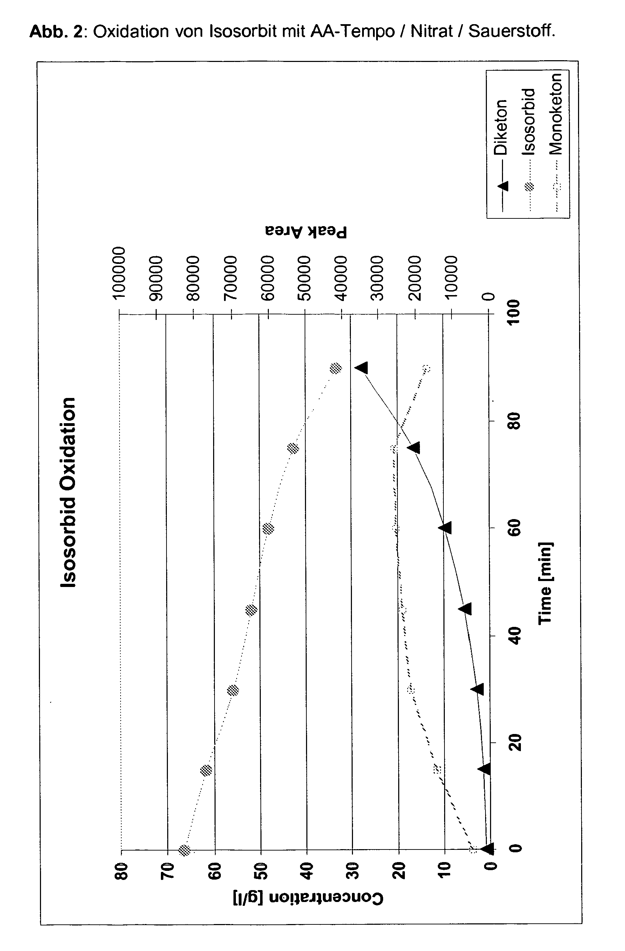

Abbildung 2 zeigt die Entwicklung der Isosorbit-, Diketon- und Monoketon-Konzentrationen in Abhängigkeit von der Zeit bei der Durchführung des erfindungsgemäßen Verfahrens. Dabei wurden die Isosorbit- und Diketon-Konzentrationen über Kalibrierkurven und dem internen Standard ausgewertet. Die Auswertung der Monoketon-Konzentrationen erfolgte über die Peakflächen.

### 7. Oxidation von Isomannit mit AA-Tempo/O₂ /Nitrit/Nitrat:

### Material:

| | | |
|---|---|---|
| Isomannit (1,4:3,6-Dianhydro-D-mannitol) | 25 mmol | → 73,07 g/L |
| AA-TEMPO | 5 mol-% | → 5,35 g/L |
| NaNO₂ | 2 mol-% | → 2,55 g/L |
| Mg(NO₃)₂ x 6H₂O | 2 mol-% | → 0,70 g/L |
| Essigsäure (100 %) | 50 mL | |
| Dekan (interner Standard für Analytik) | 1,75g | → 35 g/L |

### Reaktionsbedingungen:

| | |
|---|---|
| Reaktionstemperatur: | T = 50 °C |
| Reaktionszeit: | t = 1,5 h |
| Sauerstoff: | 10 mL / min |

### Durchführung:

Das Edukt, der Katalysator, die Salze und der interne Standard werden vorgelegt, in der Essigsäure (Zugabe der Essigsäure bis das Volumen von 50 ml erreicht ist) gelöst und in einen Vierhalskolben mit Rückflusskühler, Thermometer, Gaseinleitungsfritte und Septum zur Probennahme überführt.

Anschließend wird die Lösung unter Rühren auf eine Temperatur von 50 °C aufgeheizt. Zum Start der Reaktion wird Sauerstoff mit einer Durchflussrate von 10 mL / min über die Gaseinleitungsfritte in die Reaktionslösung eingeleitet. Die Reaktion wird über einen Zeitraum von 1,5 h gefahren, wobei zur Aufnahme der Kinetik in Abständen von 15 min eine Probe entnommen wird.

### Ergebnis:

Es wird ein Umsatz von 100 % beobachtet bei einer Selektivität für das Diketon von 100 %.

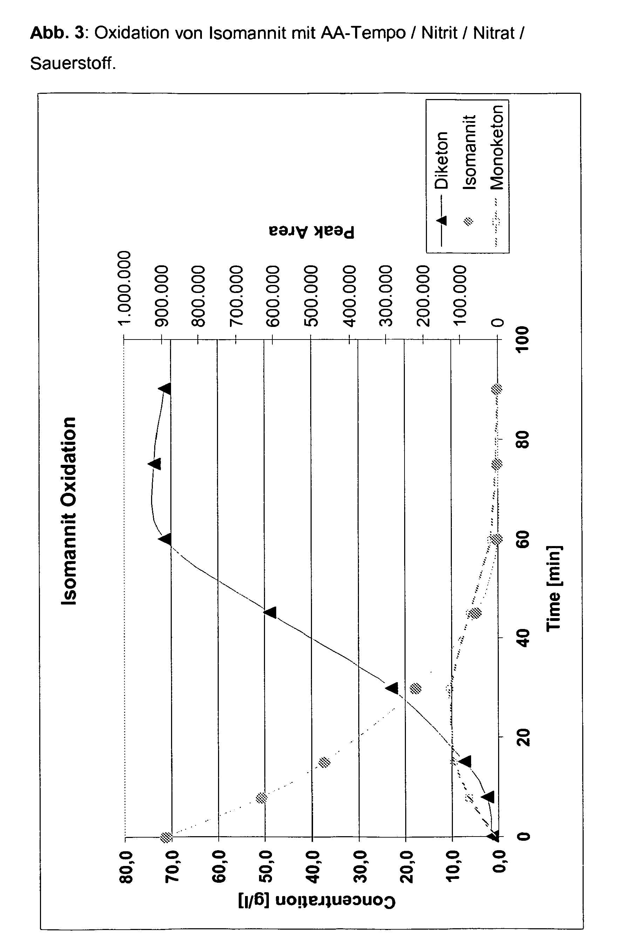

Abbildung 3 zeigt die Entwicklung der Isomannit-, Diketon- und Monoketon-Konzentrationen in Abhängigkeit von der Zeit bei der Durchführung des erfindungsgemäßen Verfahrens. Dabei wurden die Isomannit- und Diketon-Konzentrationen über Kalibrierkurven und dem internen Standard ausgewertet. Die Auswertung der Monoketon-Konzentrationen erfolgte über die Peakflächen.

**Tab.2 Zusammenstellung der Versuche zur Oxidation von Isosorbit /Isomannit mit AA-Tempo. Reaktionstemperatur 50 °C, Reaktionszeit 1,5 h, Sauerstoff 10 ml / min**

| Beispiel | Isosorbit [g/l] | AA-Tempo [g/l] | NaNO₂ [g/l] | Mg(NO₃)₂x6H₂O [g/l] | Umsatz [%] | Selektivität (Diketon) [%] |
|---|---|---|---|---|---|---|
| 5 | 73,03 | 5,370 | 2,566 | 0,700 | > 97,0 | 100 |
| 6 | 73,53 | 5,384 | - | 1,464 | 36,0 | 84 |
| 7 | Isomannit 73,04 | 5,36 | 2,590 | 0,738 | > 99,0 | 100 |

### 8. Oxidation von Isosorbit mit verschiedenen N-Oxy-Radikalen/O₂ /Nitrit/Nitrat

### Material:

| | | |
|---|---|---|
| Isosorbit (1,4:3,6-Dianhydro-D-sorbitol) | 10 mmol | → 73,07 g/L |
| N-Oxy-Radikal | 5 mol-% | |
| NaNO₂ | 2 mol-% | → 2,55 g/L |
| Mg(NO₃)₂ x 6H₂O | 2 mol-% | → 0,70 g/L |
| Essigsäure (100 %) | 20 mL | |
| Hexadekan (interner Standard für Analytik) | 0,05g | → 2,5 g/L |

### Reaktionsbedingungen:

| | |
|---|---|
| Reaktionstemperatur: | T = 50 °C |
| Reaktionszeit: | t = 1,5 h - 4,5 h |
| Sauerstoff: | 10 mL / min |

### Durchführung:

Das Edukt, der Katalysator und die Salze werden in einem Dreihalskolben mit Rückflusskühler und Gaseinleitungsfritte vorgelegt, mit 20 ml Essigsäure versetzt und unter Rühren gelöst. Anschließend wird die Lösung unter Rühren auf eine Temperatur von 50 °C aufgeheizt. Zum Start der Reaktion wird Sauerstoff mit einer Durchflussrate von 20 mL / min über die Gaseinleitungsfritte in die Reaktionslösung eingeleitet. Die Reaktionen werden über einen Zeitraum von 1,5 h - 4,5 h gefahren, wobei zur Aufnahme der Kinetik in Abständen von 30 min eine Probe entnommen wird.

### Ergebnis:

Nach dem oben beschriebenen Protokoll wurden verschiedene N-Oxy-Radikale für die Reaktion eingesetzt. Die Reaktion wurde je nach eingesetztem N-Oxy-Radikal über einen Zeitraum von 1,5 h - 4,5 h durchgeführt.

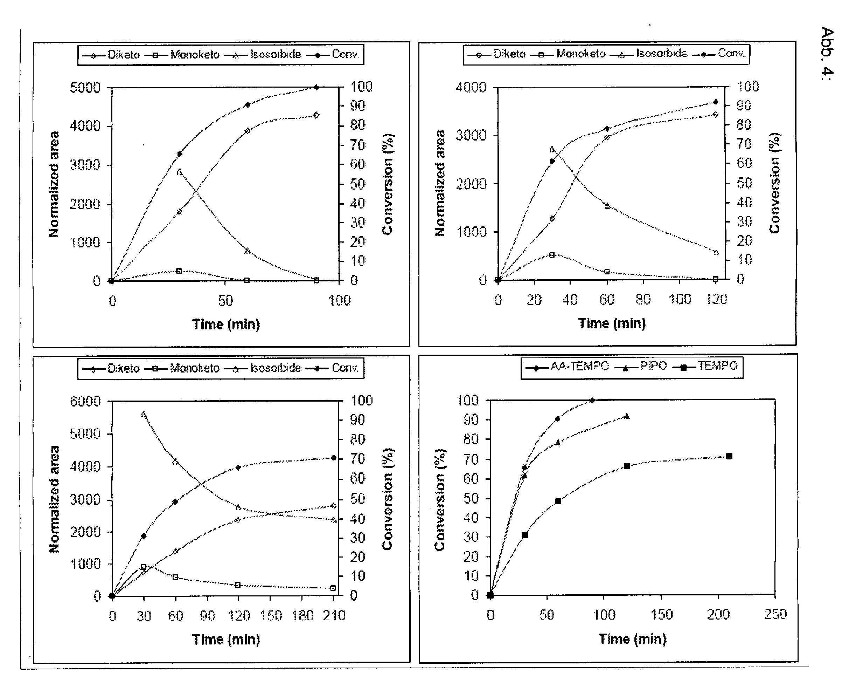

Mit dem N-Oxy-Radikal AA-Tempo konnte ein Umsatz von 100 % und eine Selektivität zum Diketon von 100 % erzielt werden. Abb. 4 zeigt die Ergebnisse der Vergleichsversuche hinsichtlich der Umsetzungen. Dargestellt wird die Oxidation von Isosorbit mit AA-Tempo (oben links), PIPO (oben rechts), Tempo (unten links) sowie ein Vergleich der Umsätze aller drei N-Oxy-Radikale in Abhängigkeit von der Zeit. Die Grafen zeigen die normalisierten Flächen der GC-Analytik und den Umsatz des Isosorbit in %, ausgewertet mit Hexadekan als internen Standard.

### 9. Oxidation von Isosorbit mit Tempo/NaClO/Bromid (Vergleichsbeispiel: Hypochloritmethode)

### Material:

| | |
|---|---|
| Isosorbit (1,4:3,6-Dianhydro-D-sorbitol) | → 20 g/L |
| Tempo | → 1,71 g/L |
| NaBr | → 11,30 g/L |
| NaOCl | → 81,5 g/L |
| Wasser | → 100 mL |

### Reaktionsbedingungen:

| | |
|---|---|
| Reaktionstemperatur: | T = 2-5 °C |
| Reaktionszeit: | t = 2 h |

### Durchführung:

2 g Isosorbit, 1127 mg Natriumbromid und 171 mg TEMPO werden in 100 ml Wasser suspendiert und in einem Eiswasserbad auf 0 °C gekühlt. Der kontinuierlich gemessene pH-Wert dieser Mischung wird durch Zugabe von 0,5 N Natronlauge exakt auf pH 10 eingestellt.
Der Versuch wird bei einem konstanten pH-Wert von 10 durchgeführt. Dieses wird durch die kontinuierliche Titration der entstehenden Säuren mit 0,5 N Natronlauge erreicht, wobei der pH-Wert mit einem automatischen Titrationssystem TITRINO, Firma Metrohm, Herisau (CH), konstant gehalten wird.

### Ergebnis:

Die Reaktion führt zu geringen Mengen Monoketon, das gewünschte Diketon ist nicht zu erkennen

## Patentansprüche

1. Verfahren zur Herstellung von 2,6-Dioxabicyclo-(3.3.0)-octan-4,8-dion **(I)** umfassend die Oxidation von Dianhydrohexitolen oder von entsprechenden Hydroxyketonen, mit einem sauerstoffhaltigen Gas in Gegenwart einer Katalysatorzusammensetzung umfassend mindestens ein Nitroxylradikal, wobei die Nitroxylradikale am α-C-Atom benachbart zum Stickstoffatom keine Wasserstoffatome aufweisen, eine oder mehrere NO-Quellen und mindestens eine oder mehrere Carbonsäuren oder deren Anhydride und/oder Mineralsäuren oder deren Anhydride gegebenenfalls in Gegenwart eines oder mehrerer Lösungsmittel, **dadurch gekennzeichnet, dass** die Umsetzung ohne Zusatz von Halogenquellen erfolgt.

2. Verfahren gemäß Anspruch 1, **dadurch gekennzeichnet, dass** als Nitroxylradikale 2,2,6,6-Tetramethylpiperidin-1-oxyl (TEMPO) und/oder die an Position 4 des Heterocyclus substituierten Derivate des 2,2,6,6-Tetramethylpiperidin-1-oxyls eingesetzt werden, wobei die Derivate einen oder mehrere Substituenten ausgewählt aus R¹, C₁-C₈-Amido-, Halogen, Oxy-, Hydroxy-, Amino-, Alkylamino-, Dialkylamino-, Arylamino-, Diarylamino-, Alkylcarbonyloxy-, Arylcarbonyloxy-, Alkylcarbonylamino- und Arylcarbonylaminogruppen aufweisen, worin R¹ eine (C₁-C₁₀)-Alkyl-, (C₁-C₁₀)-Alkenyl-, (C₁-C₁₀)-Alkoxy-, (C₆-C₁₈)-Aryl-, (C₇-C₁₉)-Aralkyl-, (C₆-C₁₈)-Aryl-(C₁-C₈)-Alkyl- oder (C₃-C₁₈)-Heteroarylgruppe bedeutet.

3. Verfahren gemäß Anspruch 1 oder 2, **dadurch gekennzeichnet, dass** der Anteil an Nitroxylradikal 0,001 bis 20 mol%, bezogen auf die Menge des eingesetzten Dianhydrohexitols, beträgt.

4. Verfahren gemäß einem oder mehreren der Ansprüche 1 bis 3, **dadurch gekennzeichnet, dass** als NO-Quelle Salpetersäure, salpetrige Säure, Ammoniumnitrat oder -nitrit, Alkali- oder Erdalkalinitrate oder - nitrite, stickoxidhaltige Gase oder Mischungen hieraus eingesetzt werden.

5. Verfahren gemäß einem oder mehreren der Ansprüche 1 bis 3, **dadurch gekennzeichnet, dass** der Anteil der NO-Quelle 0,001 bis 10 mol-%, bezogen auf die Menge des eingesetzten Dianhydrohexitols, beträgt.

6. Verfahren gemäß einem oder mehreren der Ansprüche 1 bis 5, **dadurch gekennzeichnet, dass** als Carbonsäure Essigsäure oder als Anhydrid Essigsäureanhydrid eingesetzt wird.

7. Verfahren gemäß einem oder mehreren der Ansprüche 1 bis 6, **dadurch gekennzeichnet, dass** der Anteil an Carbonsäure und oder des Anhydrids 0,1 bis 200 mol-%, bezogen auf die Menge an eingesetztem Dianhydrohexitol, beträgt.

8. Verfahren gemäß einem oder mehreren der Ansprüche 1 bis 7, **dadurch gekennzeichnet, dass** als Mineralsäuren H₂CO₃, H₃PO₄, HNO₃, HNO₂, H₂SO₄, H₂SO₃, H₃BO₃, oder deren Anhydride oder Mischungen hieraus eingesetzt werden.

9. Verfahren gemäß einem oder mehreren der Ansprüche 1 bis 8, **dadurch gekennzeichnet, dass** kein zusätzliches Lösemittel eingesetzt wird.

10. Verfahren gemäß einem oder mehreren der Ansprüche 1 bis 9, **dadurch gekennzeichnet, dass** das Reaktionswasser aus dem Reaktionsgemisch entfernt wird.

11. Verfahren gemäß Anspruch 10, **dadurch gekennzeichnet, dass** die Entfernung des Reaktionswassers durch Zusatz wasserabsorbierender Mittel zur Reaktionsmischung und/oder durch destillative oder extraktive Entfernung aus der Reaktionsmischung während der Reaktion erfolgt.

12. Verfahren gemäß Anspruch 10 oder 11, **dadurch gekennzeichnet, dass** Wasser komplexierende Lösungsmittel oder Lösungsmittel, die Wasser chemisch binden, eingesetzt werden.

13. Verfahren gemäß einem oder mehreren der Ansprüche 1 bis 12, **dadurch gekennzeichnet, dass** in Folgeschritten die Carbonsäure und ggf. das Lösungsmittel durch Destillation oder Extraktion entfernt wird, die Nitroxylradikale extrahiert und gegebenenfalls. rezykliert werden, gegebenenfalls die aus den NO-Quellen stammenden Salze entfernt werden und/oder das 2,6-Dioxabicyclo-(3.3.0)-octan-4,8-dion **(I)** durch Kristallisation, Destillation, Extraktion und/oder chromatographische Trennung gereinigt wird.

14. Verfahren gemäß einem oder mehreren der Ansprüche 1 bis 13, **dadurch gekennzeichnet, dass** das 2,6-Dioxabicyclo-(3.3.0)-octan-4,8-dion **(I)** anschließend reduktiv aminiert oder hydrocyaniert wird.

15. Verfahren gemäß Anspruch 14, **dadurch gekennzeichnet, dass** die reduktive Aminierung in zwei Stufen erfolgt, wobei a) das 2,6-Dioxabicyclo-(3.3.0)-octan-4,8-dion **(I)** in Gegenwart von Ammoniak in das entsprechende Diimin umgewandelt und b) anschließend hydriert wird.

## Claims

1. Process for preparing 2,6-dioxabicyclo[3.3.0]-octane-4,8-dione (I) comprising the oxidation of dianhydrohexitols or of corresponding hydroxyketones with an oxygen-containing gas in the presence of a catalyst composition comprising at least one nitroxyl radical, wherein the nitroxyl radicals have no hydrogen atoms on the α-C atom adjacent to the nitrogen atom, one or more NO sources and at least one or more carboxylic acids or their anhydrides and/or mineral acids or their anhydrides, optionally in the presence of one or more solvents, **characterized in that** the reaction takes place without addition of halogen sources.

2. Process according to Claim 1, **characterized in that** nitroxyl radicals used are 2,2,6,6-tetramethylpiperidine-1-oxyl (TEMPO) and/or the derivatives of 2,2,6,6-tetramethylpiperidine-1-oxyl which are substituted in position 4 of the heterocycle, the derivatives having one or more substituents selected from R¹, C₁-C₈-amido, halogen, oxy, hydroxyl, amino, alkylamino, dialkylamino, arylamino, diarylamino, alkylcarbonyloxy, arylcarbonyloxy, alkylcarbonylamino and arylcarbonylamino groups, where R¹ is a (C₁-C₁₀)-alkyl, (C₁-C₁₀)-alkenyl, (C₁-C₁₀)-alkoxy, (C₆-C₁₈)-aryl, (C₇-C₁₉) -aralkyl, (C₆-C₁₈) -aryl- (C₁-C₈)-alkyl or (C₃-C₁₈)-heteroaryl group.

3. Process according to Claim 1 or 2, **characterized in that** the fraction of nitroxyl radical is 0.001 to 20 mol%, based on the amount of dianhydrohexitol used.

4. Process according to one or more of Claims 1 to 3, **characterized in that** NO sources used are nitric acid, nitrous acid, ammonium nitrate or ammonium nitrite, alkali metal or alkaline earth metal nitrates or nitrites, nitrogen oxide-containing gases or mixtures thereof.

5. Process according to one or more of Claims 1 to 3, **characterized in that** the fraction of the NO source is 0.001 to 10 mol%, based on the amount of dianhydrohexitol used.

6. Process according to one or more of Claims 1 to 5, **characterized in that** carboxylic acid used comprises acetic acid or anhydride used comprises acetic anhydride.

7. Process according to one or more of Claims 1 to 6, **characterized in that** the fraction of carboxylic acid and/or of anhydride is 0.1 to 200 mol%, based on the amount of dianhydrohexitol used.

8. Process according to one or more of Claims 1 to 7, **characterized in that** mineral acids used are H₂CO₃, H₃PO₄, HNO₃, HNO₂, H₂SO₄, H₂SO₃, H₃BO₃, or their anhydrides or mixtures thereof.

9. Process according to one or more of Claims 1 to 8, **characterized in that** no additional solvent is used.

10. Process according to one or more of Claims 1 to 9, **characterized in that** the water of reaction is removed from the reaction mixture.

11. Process according to Claim 10, **characterized in that** the water of reaction is removed by addition of water-absorbing agents to the reaction mixture and/or by distillative or extractive removal from the reaction mixture during the reaction.

12. Process according to Claim 10 or 11, **characterized in that** water-complexing solvents or solvents which bind water chemically are used.

13. Process according to one or more of Claims 1 to 12, **characterized in that** in subsequent steps the carboxylic acid and optionally the solvent are removed by distillation or extraction, the nitroxyl radicals are extracted and optionally recycled, optionally the salts originating from the NO sources are removed and/or the 2,6-dioxa-bicyclo[3.3.0]octane-4,8-dione (I) is purified by crystallization, distillation, extraction and/or chromatographic separation.

14. Process according to one or more of Claims 1 to 13, **characterized in that** the 2,6-dioxabicyclo-[3.3.0]octane-4,8-dione (I) is subsequently subjected to reductive amination or hydrocyanation.

15. Process according to Claim 14, **characterized in that** the reductive amination takes place in two stages, a) the 2,6-dioxabicyclo[3.3.0]octane-4,8-dione (I) being converted to the corresponding diimine in the presence of ammonia and b) being subsequently hydrogenated.

## Revendications

1. Procédé de fabrication de 2,6-dioxabicyclo-(3.3.0)-octane-4,8-dione (I) comprenant l'oxydation de dianhydrohexitols ou d'hydroxycétones correspondantes avec un gaz contenant de l'oxygène en présence d'une composition catalytique comprenant au moins un radical nitroxyle, les radicaux nitroxyle ne comprenant aucun atome d'hydrogène sur l'atome C α voisin de l'atome d'azote, une ou plusieurs sources de NO et au moins un ou plusieurs acides carboxyliques ou leurs anhydrides et/ou acides minéraux ou leurs anhydrides, éventuellement en présence d'un ou de plusieurs solvants, **caractérisé en ce que** la réaction a lieu sans ajout de sources d'halogène.

2. Procédé selon la revendication 1, **caractérisé en ce que** le 2,2,6,6-tétraméthylpipéridine-1-oxyle (TEMPO) et/ou les dérivés de 2,2,6,6-tétraméthylpipéridine-1-oxyle substitués en position 4 de l'hétérocycle sont utilisés en tant que radicaux nitroxyle, les dérivés comprenant un ou plusieurs substituants choisis parmi les groupes R¹, amido en C₁-C₈, halogène, oxy, hydroxy, amino, alkylamino, dialkylamino, arylamino, diarylamino, alkylcarbonyloxy, arylcarbonyloxy, alkylcarbonylamino et arylcarbonylamino, R¹ signifiant un groupe alkyle en (C₁-C₁₀), alcényle en (C₁-C₁₀), alcoxy en (C₁-C₁₀), aryle en (C₆-C₁₈), aralkyle en (C₇-C₁₉), aryle en (C₆-C₁₈)-alkyle en (C₁-C₈) ou hétéroaryle en (C₃-C₁₈).

3. Procédé selon la revendication 1 ou 2, **caractérisé en ce que** la proportion de radical nitroxyle est de 0,001 à 20 % en moles par rapport à la quantité de dianhydrohexitol utilisé.

4. Procédé selon une ou plusieurs des revendications 1 à 3, **caractérisé en ce que** de l'acide nitrique, de l'acide nitreux, du nitrate ou nitrite d'ammonium, des nitrates ou nitrites alcalins ou alcalino-terreux, des gaz contenant de l'oxyde d'azote ou leurs mélanges sont utilisés en tant que source de NO.

5. Procédé selon une ou plusieurs des revendications 1 à 3, **caractérisé en ce que** la proportion de la source de NO est de 0,001 à 10 % en moles par rapport à la quantité de dianhydrohexitol utilisé.

6. Procédé selon une ou plusieurs des revendications 1 à 5, **caractérisé en ce que** de l'acide acétique est utilisé en tant qu'acide carboxylique ou de l'anhydride de l'acide acétique en tant qu'anhydride.

7. Procédé selon une ou plusieurs des revendications 1 à 6, **caractérisé en ce que** la proportion d'acide carboxylique et/ou de l'anhydride est de 0,1 à 200 % en moles par rapport à la quantité de dianhydrohexitol utilisé.

8. Procédé selon une ou plusieurs des revendications 1 à 7, **caractérisé en ce qu'** H₂CO₃, H₃PO₄, HNO₃, HNO₂, H₂SO₄, H₂SO₃, H₃BO₃ ou leurs anhydrides ou leurs mélanges sont utilisés en tant qu'acides minéraux.

9. Procédé selon une ou plusieurs des revendications 1 à 8, **caractérisé en ce qu'**aucun solvant supplémentaire n'est utilisé.

10. Procédé selon une ou plusieurs des revendications 1 à 9, **caractérisé en ce que** l'eau de réaction est éliminée du mélange réactionnel.

11. Procédé selon la revendication 10, **caractérisé en ce que** l'élimination de l'eau de réaction a lieu par ajout d'agents absorbant l'eau au mélange réactionnel et/ou par élimination par distillation ou par extraction à partir du mélange réactionnel pendant la réaction.

12. Procédé selon la revendication 10 ou 11, **caractérisé en ce que** des solvants qui complexent l'eau ou des solvants qui se lient chimiquement à l'eau sont utilisés.

13. Procédé selon une ou plusieurs des revendications 1 à 12, **caractérisé en ce que**, lors d'étapes ultérieures, l'acide carboxylique et éventuellement le solvant sont éliminés par distillation ou extraction, les radicaux nitroxyle sont extraits et éventuellement recyclés, les sels issus des sources de NO sont éventuellement éliminés et/ou la 2,6-dioxabicyclo-(3.3.0)-octane-4,8-dione (I) est purifiée par cristallisation, distillation, extraction et/ou séparation chromatographique.

14. Procédé selon une ou plusieurs des revendications 1 à 13, **caractérisé en ce que** la 2,6-dioxabicyclo-(3.3.0)-octane-4,8-dione (I) est ensuite aminée par réduction ou hydrocyanée.

15. Procédé selon la revendication 14, **caractérisé en ce que** l'amination réductrice a lieu en deux étapes, a) la 2,6-dioxabicyclo-(3.3.0)-octane-4,8-dione (I) étant transformée en la diimine correspondante en présence d'ammoniac, puis b) hydrogénée.
